(19) Europäisches Patentamt
European Patent Office
Office européen des brevets

(11) Numéro de publication: **0 145 554**
**B1**

(12) # FASCICULE DE BREVET EUROPÉEN

(45) Date de publication du fascicule du brevet: **25.01.89**

(51) Int. Cl.⁴: **C 07 B 39/00**, C 07 C 49/227, C 07 C 69/73

(21) Numéro de dépôt: **84402296.2**

(22) Date de dépôt: **13.11.84**

(54) **Procédé de préparation de dérivés éthyléniques chlorés.**

(30) Priorité: **18.11.83 FR 8318390**

(43) Date de publication de la demande: **19.06.85 Bulletin 85/25**

(45) Mention de la délivrance du brevet: **25.01.89 Bulletin 89/4**

(84) Etats contractants désignés: **AT BE CH DE FR GB IT LI LU NL SE**

(56) Documents cité:
**EP-A-0 082 782**
**FR-A-1 428 364**
**GB-A-1 079 421**
**US-A-2 995 600**

**ULLMANNS ENCYCLOPADIE DER TECHNISCHEN CHEMIE, 4 AUFLAGE, BAND 16, PAGE 494, VERLAG CHEMIE, WEINHEIM, NEW YORK**

**Le dossier contient des informations techniques présentées postérieurement au dépôt de la demande et ne figurant pas dans le présent fascicule.**

(73) Titulaire: **RHONE- POULENC SANTE, 20, avenue Raymond Aron, F-92160 Antony (FR)**

(72) Inventeur: **Chabardes, Pierre, 24 rue Jeanne d'Arc, F-69110 Sainte Foy Les Lyon (FR)**
Inventeur: **Mignani, Gérard, 2 avenue des Frères Lumière, F-69008 Lyon (FR)**
Inventeur: **Morel, Didier, 3 rue Jean Jaurès, F-91700 Villiers sur Orge (FR)**

(74) Mandataire: **Pilard, Jacques, RHONE- POULENC RECHERCHES Service Brevets Pharma 25, Quai Paul Doumer, F-92408 Courbevoie Cedex (FR)**

**EP 0 145 554 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne un nouveau procédé de préparation de dérivés éthyléniques chlorés de formule générale:

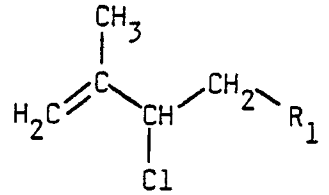

(I)

dans laquelle $R_1$ représente un atome d'halogène ou un radical acétyle, formyle éventuellement sous forme d'acétal, hydroxy éventuellement sous forme d'éther ou d'ester, alcoyloxycarbonyle, dont la partie alcoyle contient 1 à 4 atomes de carbone, alcoyle contenant 1 à 12 atomes de carbone substitué par un ou plusieurs radicaux, identiques ou différents, choisis parmi les atomes d'halogène et les radicaux acétyle, formyle éventuellement sous forme d'acétal, hydroxy éventuellement sous forme d'éther ou d'ester ou alcoyloxycarbonyle dont la partie alcoyle contient 1 à 4 atomes de carbone, alcényle contenant 2 à 12 atomes de carbone et une ou plusieurs doubles liaisons éventuellement substitué par un ou plusieurs radicaux, identiques ou différents, choisis parmi les atomes d'halogène et les radicaux acétyle, formyle éventuellement sous forme d'acétal, hydroxy éventuellement sous forme d'éther ou d'ester ou alcoyloxycarbonyle dont la partie alcoyle contient 1 à 4 atomes de carbone, $R_1$ pouvant en outre représenter un radical sulfolène-3 yle ou un radical de formule générale

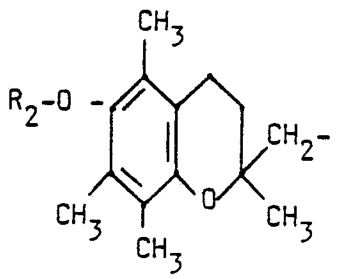

(II)

dans laquelle $R_2$ représente un atome d'hydrogène ou un radical acétyle.

Il est connu, par exemple d'après J. Mathieu et al, "L'aménagement fonctionnel en synthèse organique", Hermann (1977), p. 237 et suivantes, 242 et suivantes, 281 et suivantes, qu'à partir de molécules comportant des groupements électro-attracteurs, tel qu'un groupement carbonyle, ou des doubles liaisons isolées ou conjuguées, l'halogénation par divers agents halogénants conduit, selon les cas, soit à une substitution en $\alpha$ du groupement électro-attracteur, soit à une addition sur les doubles liaisons.

Cependant, il est connu de préparer sélectivement des dérivés allyliques halogénés à partir de composés complexes, c'est-à-dire de composés fonctionnalisés ou comportant plusieurs doubles liaisons, par action de l'acide hypochloreux par exemple sur le $\gamma$-géranate de méthyle, le myrcène, le citral sous forme d'énol ou le farnésol [cf. S.G. Hedge et coll., Tetrahedron Letters, 21, 441 (1980) et S.G. Hedge et coll., Tetrahedron, 22, 5019 (1981)] ou par action du chlore gazeux en présence d'une base minérale telle que le carbonate ou le bicarbonate de sodium en opérant dans un solvant organique tel que le tétrachlorure de carbone (cf. par exemple le brevet américain US-2 995 600).

Cependant ces procédés sont de mise en oeuvre industrielle difficile et nécessitent souvent une technologie complexe.

Il a maintenant été trouvé, et c'est ce qui fait l'objet de la présente invention, que la chloration d'un produit de formule générale

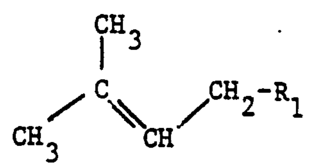

(III)

dans laquelle $R_1$ est défini comme précédemment, au moyen de chlore en opérant dans un solvant organique aprotique apolaire, conduit sélectivement au produit de formule générale (I) quélle que soit la nature du radical $R_1$.

La mise en oeuvre du procédé selon l'invention consiste à faire passer un courant de chlore gazeux éventuellement dilué dans un gaz vecteur inerte tel que l'azote ou l'argon dans une solution d'un produit de formule générale (III) dans un solvant organique anhydre convenable.

Comme solvants organiques qui conviennent particulièrement bien peuvent être cites les hydrocarbures saturés aliphatiques, par exemple le butane, le pentane ou l'hexane, les hydrocarbures aliphatiques halogénés, par exemple le chlorure de méthylène, les hydrocarbures cycloaliphatiques, par exemple le cyclopentane ou le cyclohexane ou les hydrocarbures aromatiques, par exemple le benzène ou le toluène.

Le procédé est généralement mis en oeuvre à une température comprise entre -10 et 100°C et, de préférence entre 10 et 50°C. Il est avantageux d'opérer à la température d'ébullition du solvant éventuellement sous pression réduite. Cette manière d'opérer facilite l'élimination de l'acide chlorhydrique formé au cours de la réaction. Il ést également possible de distiller le solvant, tout en introduisant du solvant frais afin d'opérer à volume constant, le solvant récupéré pouvant être recyclé dans des opérations ultérieures, après lavage pour éliminer l'acide chlorhydrique et séchage.

La concentration du produit de formule générale (II) dans le solvant n'est pas critique et elle est généralement de 1 partie en poids de produit de formule générale (II) pour 1 à 4 parties de solvant.

Les produits de formule générale (I) peuvent être séparés du mélange réactionnel après évaporation totale du solvant éventuellement lavé pour éliminer l'acide chlorhydrique formé.

Les produits de formule générale (I) ainsi obtenus peuvent être purifiés par application des méthodes habituelles telles que la cristallisation, la distillation ou la chromatographie.

Les produits de formule générale (I) dans laquelle $R_1$ représente un radical oxo-2 carbométhoxy-1 propyle, oxo-6 méthyl-2 heptène-2 yle, oxo-6 carbométhoxy-5 méthyl-2 heptène-2 yle, oxo-6 méthylène-2 heptyle, oxo-6 méthylène-2 carbométhoxy-5 heptyle, diméthyl-2,6 oxo-10 undécadiène-2,6 yle, diméthyl-2,6 carbométhoxy-9 oxo-10 undécadiène-2,6 yle, méthyl-2 méthylène-6 oxo-10 undécène-2 yle, méthyl-2 méthylène-6 carbométhoxy-9 oxo-10 undécène-2 yle éventuellement sous forme d'acétal, sont des produits nouveaux qui constituent un autre objet de la présente invention.

Le procédé selon la présente invention permet d'obtenir les produits de formule générale (I) avec des rendements généralement supérieurs à 70 % par la mise en oeuvre de techniques industrielles plus simples que celles nécessaires à la réalisation des procédés antérieurement connus.

Les produits obtenus selon le procédé de la présente invention constituent des intermédiaires particulièrement utiles pour la préparation de produits terpéniques tels que la vitamine E. D'un intérêt tout particulier sont la chloro-3 méthylhepténone (chloro-3 méthyl-2 heptène-1 one-6), la chloro-3 géranylacétone (diméthyl-2,6 chloro-3 undécadiène-1,6 one-10), la chloro-3 farnésylacétone (triméthyl-2,6,10 chloro-3 pentadécatriène-1,6,10 one-14) et le chloro-3 myrcène (chloro-3 méthyl-2 méthylène-6 octadiène-1,7).

Les exemples suivants, donnés à titre non limitatif, montrent comment l'invention peut être mise en pratique.

**Exemple 1**

Dans un ballon de 500 $cm^3$, on introduit, sous atmosphère d'argon, 58,96 g de géranylacétone, mélange cis-trans [$(CH_3)_2C=CH-CH_2CH_2-C(CH_3)=CH-CH_2CH_2-CO-CH_3$ soit 0,3031 mole, et 200 $cm^3$ de pentane sec. On chauffe à reflux puis on introduit dans cette solution 21,5 g de chlore gazeux (0,3 mole) en 1 heure 50 minutes. Après évaporation du solvant, on obtient 75,08 g d'une huile jaune qui, par distillation à 119-124°C sous une pression de 0,02 kPa, fournit 35,98 g de chloro-3 géranylacétone de formule: $CH_3C(=CH_2)-CHCl-CH_2-CH_2-C(CH_3)=CH-CH_2-CH_2-CO-CH_3$ soit 0,157 mole.

Le rendement en produit isolé est de 51,8 % par rapport à la géranylacétone mise en oeuvre.

Le dosage, par chromatographie en phase gazeuse du produit brut de la réaction, montre que:

- le taux de transformation de la géranylacétone est de 88,5
- le rendement en chloro-3 géranylacétone est de 64,9 % par rapport à la geranylacétone mise en oeuvre
- le rendement en chloro-3 géranylacétone est de 73,3 % par rapport à la géranylacétone transformée.

La structure du produit obtenu est confirmée par le spectre infra-rouge et le spectre de masse. Le spectre de résonance magnétique nucléaire du proton montre que le produit obtenu est constitué de 60 % d'isomère trans et de 40 % d'isomère cis.

**Exemple 2**

Dans un ballon tricol de 500 $cm^3$, on introduit, sous atmosphère d'argon 25 g (128,8 m.moles) d'un mélange équimoléculaire des deux produits isomères de formule:

$(CH_3)_2C=CH-CH_2CH_2-C(CH_3)=CH-CH_2CH_2-CO-CH_3$ et
$(CH_3)_2C=CH-CH_2CH_2-C(=CH_2)-CH_2CH_2CH_2CO\ CH_3$

et 100 $cm^3$ de pentane sec. On chauffe à reflux puis on introduit en 1 heure 8,87 g de chlore gazeux (125 m.moles). Après évaporation du solvant, on obtient 32,54 g d'une huile jaune qui, par distillation à 121-126°C

sous une pression de 0,02 kPa fournit 17,5 g (76 m.moles) d'un mélange 25-75 des produits de formule:
$CH_2=C(CH_3)-CHCl-CH_2CH_2-C(CH_3)=CH-CH_2CH_2COCH_3$ et
$CH_2=C(CH_3)-CHCl-CH_2CH_2C(=CH_2)-CH_2CH_2CH_2CO\ CH_3$

Le rendement en produit isolé est de 59,5 % par rapport aux produits mis en oeuvre.
Le dosage, par chromatographie en phase gazeuse, du produit brut de la réaction montre que:
- le taux de transformation des produits mis en oeuvre est de 95,7 %
- le rendement est de 60,6 % par rapport aux produits mis en oeuvre
- le rendement est de 63,3 % par rapport aux produits mis en oeuvre transformés.

Les produits obtenus sont caractérisés par le spectre infra-rouge, le spectre de masse et le spectre de résonance magnétique nucléaire du proton.

**Exemple 3** (essai comparatif)

Dans un ballon tricol de 500 cm$^3$, on introduit, sous atmosphère d'argon, 53,74 g (0,277 mole) de géranylacétone (mélange cis-trans), 200 cm$^3$ de pentane sec et 30 g de carbonate de sodium (0,283 mole). On chauffe à reflux puis on introduit en 1 heure 30 minutes, 19,6 g de chlore gazeux (0,277 mole). Après refroidissement, le mélange réactionnel est filtré puis lavé à l'eau jusqu'à neutralité. Les phases organiques sont séchées. Après filtration sur papier-filtre (papier Whatmann), le solvant est évaporé. On obtient ainsi 67,07 g d'une huile jaune qui, après distillation sous pression réduite, fournit 23,4 g (0,102 mole) d'un mélange cis-trans du produit de formule:
$CH_2=C(CH_3)-CHCl-CH_2CH_2-C(CH_3)=CH-CH_2CH_2CO\ CH_3$.

Le rendement en produit isolé est de 37 % par rapport à la géranylacétone mise en oeuvre.
Le dosage, par chromatographie en phase gazeuse, du produit brut de la réaction montre que:
- le taux de transformation de la géranylacétone est de 88,6 %
- le rendement en chloro-3 géranylacétone est de 40,1 % par rapport à la géranylacétone mise en oeuvre
- le rendement en chloro-3 géranylacétone est de 45,2 % par rapport à la géranylacétone transformée.

La structure du produit obtenu est confirmée par le spectre infra-rouge, le spectre de masse et le spectre de résonance magnétique nucléaire du proton.

**Exemple 4**

Dans un ballon tricol de 500 cm$^3$, on introduit, sous atmosphère d'argon, 38 g (0,3 mole) de méthylhepténone [$(CH_3)_2C=CH-CH_2CH_2-CO-CH_3$] et 200 cm$^3$ de pentane sec. On chauffe au reflux puis on introduit, en 1 heure 30 minutes, 21,3 g de chlore gazeux (0,3 mole). Après refroidissement, on évapore le solvant. On obtient 52,74 g d'une huile jaune qui, par distillation à 58°C sous pression réduite (0,17 kPa), fournit 42,46 g d'un mélange contenant 35 % de chloro-3 méthylhepténone, d'après dosage par le spectre de résonance magnétique nucléaire du proton.

Le taux de transformation de la méthyl-6 heptène-5 one-2 est de 75 %.

Le rendement en chloro-3 méthyl-2 heptène-1 one-6 est de 35 % par rapport à la méthylhepténone mise en oeuvre. La sélectivité est de 46,6 %.

La structure du produit obtenu est confirmée par le spectre infra-rouge, le spectre de masse et le spectre de résonance magnétique nucléaire du proton.

**Exemple 5**

Dans un ballon tricol de 500 cm$^3$, on introduit, sous atmosphère d'argon, 77,84 g (0,297 mole) de $(CH_3)_2C=CH-CH_2CH_2-C(CH_3)=CH-CH_2CH_2-C(CH_3)=CH-CH_2CH_2-CO-CH_3$ et 200 cm$^3$ de pentane sec. On chauffe au reflux puis on introduit, en 1 heure 30 minutes, 21,3 g de chlore gazeux (0,3 mole). Après évaporation du solvant, on récupère 89,20 g d'une huile jaune qui, par distillation à 170-175°C sous pression réduite (0,053 kPa), fournit 30 g (0,101 mole) de produit de formule:
$CH_2=C(CH_3)-CHCl-CH_2CH_2C(CH_3)=CH-CH_2CH_2-C(CH_3)=CH-CH_2CH_2-CO-CH_3$
avec un rendement de 34,1 % par rapport au produit mis en oeuvre.

La structure du produit obtenu est confirmée par le spectre de masse et le spectre infra-rouge.

**Exemple 6**

Dans un ballon tricol de 1 litre, on introduit, sous atmosphère d'argon, 42 g (0,3088 mole) de myrcène distillé et 104 $cm^3$ d'hexane sec. On chauffe à 65°C, puis on introduit, en 2 heures 30 minutes, 21,6 g (0,3088 mole) de chlore gazeux tout en distillant l'hexane (400 $cm^3$), l'hexane distillé étant compensé par l'introduction, en cours de distillation, de 400 $cm^3$ d'hexane. Après évaporation du solvant sous pression réduite (2,7 kPa), on obtient 54,52 g d'une huile jaune, dont l'analyse par chromatographie en phase gazeuse montre qu'elle contient 71,3 % de chloro-3 myrcène et 10,1 % de myrcène n'ayant pas réagi.

Le taux de transformation du myrcène est de 86,8 %.

Le rendement est de 63,5 % par rapport au myrcène mis en oeuvre et de 73,2 % par rapport au myrcène transformé.

**Exemple 7**

Dans un ballon tricol de 500 $cm^3$, on introduit, sous atmosphère d'argon, 25 g d'un mélange cis-trans de

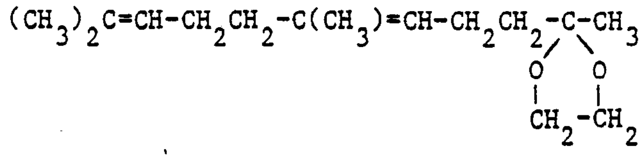

titrant 85 % soit 89,3 m.moles et 100 $cm^3$ de pentane. On chauffe au reflux puis on introduit, en 30 minutes, 89,3 m.moles de chlore gazeux. Après évaporarion du solvant, on obtient 29,2 g d'une huile jaune qui, par distillation à 135°C sous une pression de 0,12 kPa, fournit 17,68 g (64,8 m.moles) d'un mélange cis-trans de produit de formule:

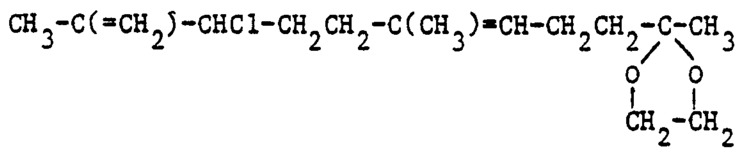

Le rendement en produit isolé est de 72,6 % par rapport à l'acétal mis en oeuvre.

La structure du produit obtenu est confirmée par le spectre infra-rouge, le spectre de masse et le specrre de résonance magnétique nucléaire du proton.

**Exemple 8**

Dans un ballon tricol de 500 $cm^3$, on introduit, sous atmosphère d'argon, 28,21 g d'un mélange constitué des produits:

$(CH_3)_2C=CH\text{-}CH_2CH_2\text{-}C(CH_3)=CH\text{-}CH_2\text{-}CH(COCH_3)\text{-}COOCH_3$ et

$(CH_3)_2C=CH\text{-}CH_2CH_2\text{-}C(=CH_2)\text{-}CH_2\text{-}CH_2\ CH(COCH_3)\ COOCH_3$

soit 112 m.moles, en 100 $cm^3$ de pentane sec. On chauffe au reflux puis on introduit, en 1 heure 30 minutes, 112 m.moles de chlore gazeux. Après évaporation du solvant, on obtient 33,62 g d'une huile jaune clair qui, par distillation à 146°C sous 0,11 kPa, fournit 27,86 g (97,2 m.moles) d'un mélange des produits de formule:

$CH_2=C(CH_3)\text{-}CHCl\text{-}CH_2CH_2\text{-}C(CH_3)=CH\text{-}CH_2\text{-}CH(COCH_3)COOCH_3$ et

$CH_2=C(CH_3)\text{-}CHCl\text{-}CH_2CH_2\text{-}C(=CH_2)\text{-}CH_2CH_2\text{-}CH(COCH_3)COOCH_3$.

Le taux de transformation des produits mis en oeuvre est de 100 %.

Le rendement est de 86,7 % par rapport aux produits mis en oeuvre.

**Exemple 9**

On utilise un réacteur de 4 litres muni d'une agitation mécanique, d'un thermomètre, d'un dispositif de barbotage de gaz, d'une arrivée de pentane (réglée par une pompe), d'une colonne de distillation dont la sortie est reliée à un ballon de 5 litres contenant de la soude 1,5 N, que l'on fait circuler au moyen d'une pompe de haut en bas d'une colonne garnie d'anneaux de Raschig surmontant le ballon (afin d'éliminer l'acide chlorhydrique entraîné par le pentane), et relié lui-même à un ballon dans lequel le pentane est condensé.

Dans le réacteur, on introduit, sous atmosphère d'argon, 840 g (6,176 moles) de myrcène pur et 2 litres de pentane. On chauffe le ballon au moyen d'un bain à 45-55°C. La température du mélange réactionnel est de 35°C. On fait alors passer un mélange gazeux de chlore et d'argon, le rapport des débits de chlore et d'argon

étant 7/12. On élimine par distillation le pentane et l'acide chlorhydrique formé tout en ajoutant simultanément du pentane afin de maintenir un volume constant. La vitesse d'addition du chlore est de 167 g/heure et celle du pentane de 2 litres/heure. Au bout de 2 heures 40 minutes, on a introduit 440 g de chlore (6,19 moles) et 5 litres de pentane. Le volume de pentane recueilli est de 5,5 litres. Après la fin de l'addition du chlore, le pentane contenu dans le réacteur est éliminé par distillation à 40° C sous 5,3 kPa. On obtient ainsi un résidu pesant 1080 g qui est distillé rapidement entre 34 et 60° C sous 0,08 kpa. Le distillat obtenu (1026 g) titre 86,9 % en chloro-3 myrcène. Le rendement est de 84,6 % par rapport au myrcéne mis en oeuvre.

Le dosage du myrcène dans les distillats légers montre que le taux de conversion est de 96,4 %.

Le rendement est de 87,8 % par rapport au myrcéne ayant réagi.

**Exemple 10**

Dans un ballon tricol de 1 litre, on introduit, sous atmosphère d'argon, 77,42 g de linalol (0,5 mole) de formule $(CH_3)_2CH=CH\text{-}CH_2CH_2\text{-}C(CH_3)(OH)\text{-}CH=CH_2$, et 600 $cm^3$ de pentane sec. On chauffe au reflux puis on introduit, en 2 heures, 35,5 g de chlore gazeux (0,5 mole).

Après évaporation du solvant, on récupère 97,74 g d'une huile incolore contenant le produit de formule:

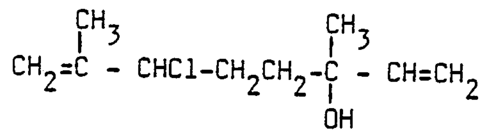

Ce produit est obtenu avec un rendement de 55 % par rapport au linalol mis en oeuvre.

**Exemple 11**

Dans un ballon de 500 $cm^3$, on introduit, sous atmosphère d'argon, 250 $cm^3$ de chlorure de méthylène et 7,9 g de tétraméthyl-2,5,7,8 (méthyl-4 pentène-3 yl)-2 chromanol-6 titrant 90 % soit 24,68 m.moles. On chauffe au reflux puis on introduit, en 1 heure, 2 g de chlore gazeux (28,16 m.moles). Après évaporation du solvant, on obtient 8,62 g d'une huile légèrement jaune dont l'analyse par chromatographie en phase liquide montre qu'elle contient 57,7 % de tétraméthyl-2,5,7,8 (chloro-3 méthyl-4 pentène-4 yl)-2 chromanol-6.

Le taux de transformation du produit de départ est de 71 %. Le rendement est de 81 % par rapport au produit de départ transformé.

La structure du produit obtenu est confirmée par le spectre infra-rouge, le spectre de masse et le spectre de résonance magnétique nucléaire du proton.

**Exemple 12**

Dans un réacteur tricol de 250 $cm^3$ muni d'une agitation mécanique, d'une arrivée plongeante de gaz, d'une ampoule de coulée, d'un dispositif d'évacuation des gaz et de piégeage du pentane distillé, on introduit 29,4 g d'acétate de linalyle (0,15 mole) et 60 $cm^3$ de pentane sec. On chauffe à 36° C puis on introduit du chlore gazeux dilue par de l'argon, le pentane distillé étant remplacé par du pentane frais de façon à maintenir le niveau constant dans le réacteur. Après 2 heures 18 minutes, on a distillé et introduit 300 $cm^3$ de pentane et on a ajouté 11,3 g de chlore (0,159 mole). Après évaporation du solvant sous pression réduite (20 mm de mercure; 2,7 kPa) on obtient 34,5 g d'une huile légèrement jaune.

L'analyse par chromatographie en phase gazeuse montre que l'huile obtenue contient 86 % d'acétoxy-6 chloro-3 diméthyl-2,6 octadiène-1,7.

Le taux de transformation de l'acétate de linalyle est de 100 %.

La structure du produit obtenu est confirmée par le spectre de résonance magnétique nucléaire du proton qui montre la présence d'un mélange équimoléculaire des 2 diastéréoisomères.

**Exemple 13**

On opère dans un appareil identique à celui décrit dans l'exemple 12. On introduit 25,9 g de chlorure de géranyle (0,15 mole) et 60 $cm^3$ de pentane sec. On chauffe le mélange réactionnel à 37° C puis on introduit du chlore gazeux dilué par de l'argon, le pentane distillé étant remplacé par du pentane frais de façon à maintenir le niveau constant dans le réacteur. Après 3 heures, on a distillé et introduit 360 $cm^3$ de pentane et on a ajouté

10,6 g de chlore (0,15 mole). Après évaporation du solvant, on obtient 30,6 g d'un produit brut qui est distillé rapidement à 95° C sous pression réduite (0,5 mm de mercure; 0,067 kPa).

L'analyse du distillat par chromatographie en phase gazeuse et par le spectre de résonance magnétique nucléaire du proton montre que le taux de transformation du chlorure de géranyle est de 65 % et que le dichloro-3,8 diméthyl-2,6 octadiène-1,6 est obtenu avec un rendement de 91 % par rapport au chlorure de géranyle transformé.

**Revendications** pour les Etats contractants BE, CH, DE, FR, GB, IT, LI, LU, NL, SE

1. Procédé de préparation de dérivés éthyléniques chlorés de formule générale:

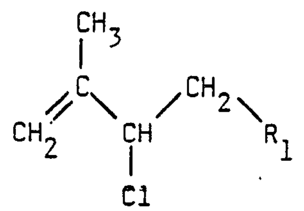

(I)

dans laquelle R[1] représente un atome d'halogène ou un radical acétyle, formyle éventuellement sous forme d'acétal, hydroxy éventuellement sous forme d'éther ou d'ester, alcoyloxycarbonyle dont la partie alcoyle contient 1 à 4 atomes de carbone, alcoyle contenant 1 à 12 atomes de carbone substitué par un ou plusieurs radicaux, identiques ou différents, choisis parmi les atomes d'halogène et les radicaux acétyle, formyle éventuellement sous forme d'acétal, hydroxy éventuellement sous forme d'éther ou d'ester, ou alcoyloxycarbonyle dont la partie alcoyle contient 1 à 4 atomes de carbone, alcényle contenant 2 à 12 atomes de carbone et une ou plusieurs doubles liaisons éventuellement substitué par un ou plusieurs radicaux, identiques ou différents, choisis parmi les atomes d'halogène et les radicaux acétyle, formyle éventuellement sous forme d'acétal, hydroxy éventuellement sous forme d'éther ou d'ester ou alcoyloxycarbonyle dont la partie alcoyle contient 1 à 4 atomes de carbone, $R_1$ pouvant en outre représenter un radical sulfolène-3 yle ou un radical de formule générale:

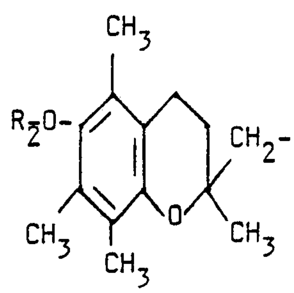

(II)

dans laquelle $R_2$ représente un atome d'hydrogène ou un radical acétyle, caractérisé en ce que l'on effectue la chloration d'un produit de formule generale:

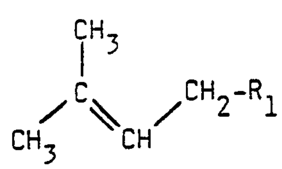

III

au moyen de chlore en opérant dans un solvant organique aprotique apolaire à une température comprise entre -10 et 100° C.

2. Procédé selon la revendication 1 caractérisé en ce que le solvant est choisi parmi les hydrocarbures saturés aliphatiques, les hydrocarbures aliphatiques halogénés, les hydrocarbures cycloaliphatiques et les hydrocarbures aromatiques.

3. Procédé selon la revendication 1 caractérisé en ce que l'on opère à la température de reflux du mélange réactionnel éventuellement sous pression réduite.

4. Procédé selon la revendication 1 caractérisé en ce qué l'on distille le solvant en opérant à volume constant.

5. Les nouveaux dérivés éthyléniques chlorés de formule générale:

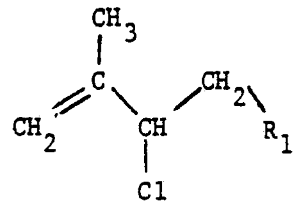 (I)

dans laquelle $R_1$ représente un radical oxo-2 carbométhoxy-1 propyle, oxo-6 méthyl-2 heptène-2 yle, oxo-6 carbométhoxy-5 méthyl-2 heptène-2 yle, oxo-6 méthylène-2 heptyle, oxo-6 méthylène-2 carbométhoxy-5 heptyle, diméthyl-2,6 oxo-10 undécadiène-2,6 yle, diméthyl-2,6 carbométhoxy-9 oxo-10 undécadiène-2,6 yle, méthyl-2 méthylène-6 oxo-10 undécène-2 yle et méthyl-2 méthylène-6 carbométhoxy-9 oxo-10 undécène-2 yle éventuellement sous forme d'acétal.

**Revendications** pour l'Etat contractant AT

1. Procédé de préparation de dérivés éthyléniques chlorés de formule générale:

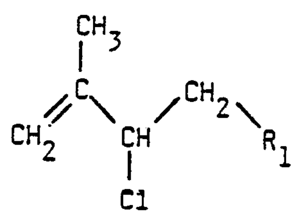 (I)

dans laquelle $R_1$ représente un atome d'halogène ou un radical acétyle, formyle éventuellement sous forme d'acétal, hydroxy éventuellement sous forme d'éther ou d'ester, alcoyloxycarbonyle dont la partie alcoyle contient 1 à 4 atomes de carbone, alcoyle contenant 1 à 12 atomes de carbone substitué par un ou plusieurs radicaux, identiques ou différents, choisis parmi les atomes d'halogène et les radicaux acétyle, formyle éventuellement sous forme d'acétal, hydroxy éventuellement sous forme d'éther ou d'ester, ou alcoyloxycarbonyle dont la partie alcoyle contient 1 à 4 atomes de carbone, alcényle contenant 2 à 12 atomes de carbone et une ou plusieurs doubles liaisons éventuellement substitué par un ou plusieurs radicaux, identiques ou différents, choisis parmi les atomes d'halogène et les radicaux acétyle, formyle éventuellement sous forme d'acétal, hydroxy éventuellement sous forme d'éther ou d'ester ou alcoyloxycarbonyle dont la partie alcoyle contient 1 à 4 atomes de carbone, $R_1$ pouvant en outre représenter un radical sulfolène-3 yle ou un radical de formule générale:

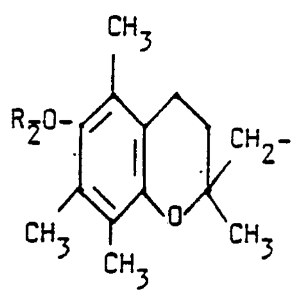 (II)

dans laquelle $R_2$ représente un atome d'hydrogène ou un radical acétyle, caractérisé en ce que l'on effectue la chloration d'un produit de formule générale:

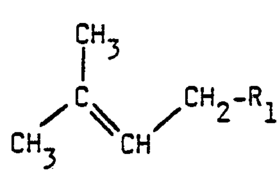 (III)

au moyen de chlore en opérant dans un solvant organique aprotique apolaire à une température comprise entre -10 et 100° C.

2. Procédé selon la revendication 1 caractérisé en ce que le solvant est choisi parmi les hydrocarbures saturés aliphatiques, les hydrocarbures aliphatiques halogénés, les hydrocarbures cycloaliphatiques et les hydrocarbures aromatiques.

3. Procédé selon la revendication 1 caractérisé en ce que l'on opère à la température de reflux du mélange réactionnel éventuellement sous pression réduite.

4. Procédé selon la revendication 1 caractérisé en ce que l'on distille le solvant en opérant à volume constant.

**Patentansprüche** für die Vertragsstaaten BE, CH, DE, FR, GB, IT, LI, LU, NL, SE

1. Verfahren zur Herstellung von chlorierten ethylenischen Derivaten der allgemeinen Formel:

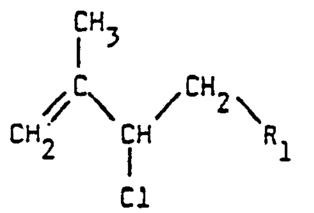

(I)

worin $R_1$ bedeutet ein Halogenatom oder einen Acetylrest, in Ether- oder Esterform Alkyloxycarbonyl, dessen Alkylteil 1 bis 4 Kohlenstoffatome enthält, Alkyl mit 1 bis 12 Kohlenstoffatomen, substituiert durch einen oder mehrere Reste, die identisch oder verschieden sind, ausgewählt unter den Halogenatomen und den Acetylresten, Formyl, gegebenenfalls in Acetalform, Hydroxy, gegebenenfalls in Ether- oder Esterform, oder Alkyloxycarbonyl, dessen Alkylteil 1 bis 4 Kohlenstoffatome enthält, Alkenyl, enthaltend 2 bis 12 Kohlenstoffatome und eine oder mehrere Doppelbindungen, gegebenenfalls substituiert durch einen oder mehrere Reste, die identisch oder verschieden sind, ausgewählt unter den Halogenatomen und den Resten Acetyl, Formyl, gegebenenfalls in Acetalform, Hydroxy, gegebenenfalls in Ether- oder Esterform, oder Alkyloxycarbonyl, dessen Alkylteil 1 bis 4 Kohlenstoffatome enthält, wobei $R_1$ außerdem einen Sulfolen-3-yl-Rest oder einen Rest der allgemeinen Formel:

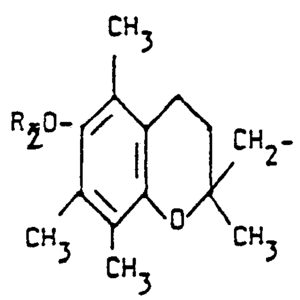

(II)

bedeuten kann, worin $R_2$ ein Wasserstoffatom oder einen Acetylrest bedeutet,
dadurch gekennzeichnet, daß man die Chlorierung eines Produkts der allgemeinen Formel:

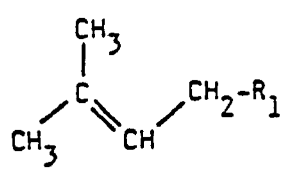

(III)

mittels Chlor durchführt, indem in einem apolaren aprotischen organischen Lösungsmittel bei einer Temperatur zwischen -10 und 100° C gearbeitet wird.

2. Verfahren gemäß Anspruch 1, dadurch gekennzeichnet, daß das Lösungsmittel ausgewählt ist unter den gesättigten aliphatischen Kohlenwasserstoffen, den halogenierten aliphatischen Kohlenwasserstoffen, den cycloaliphatischen Kohlenwasserstoffen und den aromatischen Kohlenwasserstoffen.

3. Verfahren gemäß Anspruch 1, dadurch gekennzeichnet, daß man bei der Rückflußtemperatur des Reaktionsgemisches gegebenenfalls unter vermindertem Druck arbeitet.

4. Verfahren gemäß Anspruch 1, dadurch gekennzeichnet, daß man das Lösungsmittel abdestilliert, indem bei konstantem Volumen gearbeitet wird.

5. Die neuen chlorierten ethylenischen Derivate der allgemeinen Formel:

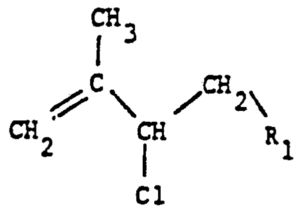

(I)

worin $R_1$ bedeutet einen 2-Oxo-1-carbomethoxypropylrest, 6-Oxo-2-methylhepten-2-yl, 6-Oxo-5-carbomethoxy-2-methylhepten-2-yl, 6-Oxo-2-methylenheptyl, 6-Oxo-2-methylen-5-carbomethoxyheptyl, 2,6-Dimethyl-10-oxo-undecadien-2,6-yl, 2,6-Dimethyl-9-carbomethoxy-10-oxo-undecadien-2,6-yl, 2-Methyl-6-methylen-10-oxo-undecen-2-yl und 2-Methyl-6-methylen-9-carbomethoxy-10-oxo-undecen-2-yl, gegebenenfalls in Acetalform.

**Patentansprüche** für den Vertragsstaat AT:

1. Verfahren zur Herstellung chlorierter Äthylenverbindungen der allgemeinen Formel:

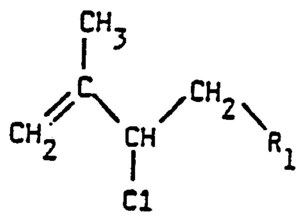

(I)

in welcher $R_1$ ein Halogenatom oder einen Acetyl-, Formyl- (gegebenenfalls in Acetalform), Hydroxy- (gegebenenfalls in Äther- oder Esterform), Alkyloxycarbonyl- (dessen Alkylteil 1 bis 4 Kohlenstoffatome enthält), Alkyl- [mit 1 bis 12 Kohlenstoffatomen und substituiert durch einen oder mehrere gleiche oder verschiedene Reste, ausgewählt aus den Halogenatomen und den Acetyl-, Formyl- (gegebenenfalls in Acetalform), Hydroxy- (gegebenenfalls in Äther- oder Esterform) oder Alkyloxycarbonylresten, deren Alkylteil 1 bis 4 Kohlenstoffatome enthält], Alkenylrest mit 2 bis 12 Kohlenstoffatomen und einer oder mehreren Doppelbindungen (gegebenenfalls substituiert durch einen oder mehrere gleiche oder verschiedene Reste, ausgewählt aus den Halogenatomen und den Acetyl-, Formyl- (gegebenenfalls in Acetalform), Hydroxy- (gegebenenfalls in Äther- oder Esterform) oder Alkyloxycarbonylresten, deren Alkylteil 1 bis 4 Kohlenstoffatome enthält] darstellt, wobei $R_1$ außerdem einen Sulfolen-3-ylrest oder einen Rest der allgemeinen Formel.

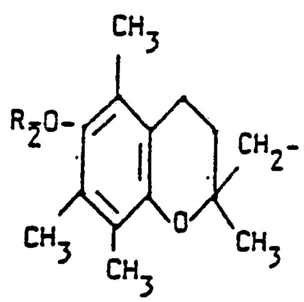

(II)

darstellen kann, in welcher $R_2$ für ein Wasserstoffatom oder einen Acetylrest steht, dadurch gekennzeichnet, daß man eine Verbindung der allgemeinen Formel:

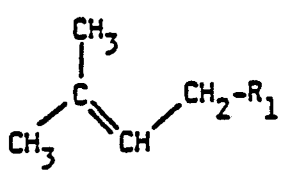

(III)

mit Chlor chloriert, indem man in einem apolaren, aprotischen, organischen Lösungsmittel bei einer Temperatur zwischen -10 und 100°C arbeitet.

2. Verfahren nach Anspruch 1, dadurch gekennzeichnet, daß das Lösungsmittel ausgewählt ist aus den aliphatischen, gesättigten Kohlenwasserstoffen, den aliphatischen Halogenkohlenwasserstoffen, den cycloaliphatischen Kohlenwasserstoffen und den aromatischen Kohlenwasserstoffen.

3. Verfahren nach Anspruch 1, dadurch gekennzeichnet, daß man bei der Rückflußtemperatur der Reaktionsmischung, gegebenenfalls unter vermindertem Druck arbeitet.

4. Verfahren nach Anspruch 1, dadurch gekennzeichnet, daß man das Lösungsmittel destilliert, indem man bei konstantem Volumen arbeitet

**Claims** for the contracting states BE, CH, DE, FR, GB, IT, LI, LU, NL, SE

1. Process for preparing chlorinated ethylenic derivatives of general formula:

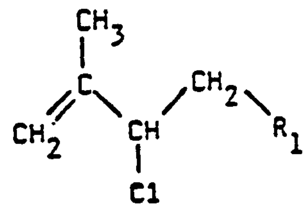

in which $R_1$ denotes a halogen atom or an acetyl or formyl radical if appropriate in the form of acetal, a hydroxy radical if appropriate in the form of ether or ester, an alkyloxycarbonyl radical whose alkyl part contains 1 to 4 carbon atoms, an alkyl radical containing from 1 to 12 carbon atoms and substituted by one or more identical or different radicals chosen from halogen atoms and acetyl and formyl radicals if appropriate in the form of acetal, a hydroxy radical if appropriate in the form of ether or ester or an alkyloxycarbonyl radical whose alkyl part contains 1 to 4 carbon atoms, an alkenyl radical containing 2 to 12 carbon atoms and one or more double bonds if appropriate substituted by one or more identical or different radicals, chosen from halogen atoms and acetyl and formyl radicals optionally in the form of acetal, a hydroxy radical if appropriate in the form of ether or ester or to 4 carbon atoms, $R_1$ being moreover able to denote a 3-sulpholenyl radical or a radical of general formula:

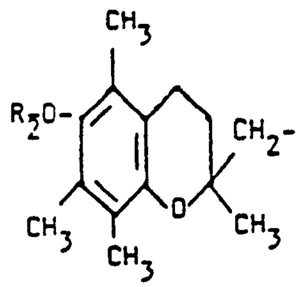

in which $R_2$ denotes a hydrogen atom or an acetyl radical, characterized in that chlorination of a product of general formula

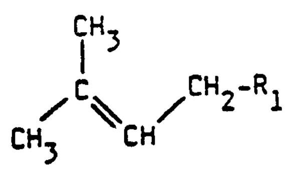

is carried out by means of chlorine by operating in a non-polar aprotic organic solvent at a temperature of between -10 and 100°C.

2. Process according to Claim 1, characterised in that the solvent is chosen from aliphatic saturated hydrocarbons, halogenated aliphatic hydrocarbons, cycloaliphatic hydrocarbons and aromatic hydrocarbons.

3. Process according to Claim 1, characterized in that it comprises operating at the reflux temperature of the reaction mixture, under reduced pressure if appropriate.

4. Process according to Claim 1, characterized in that the solvent is distilled off in an operation at constant volume.

5. New chlorinated ethylenic derivatives of general formula:

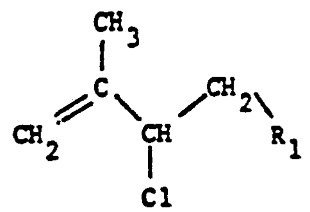

in which $R_1$ denotes a 2-oxo-1-carbomethoxypropyl, 6-oxo-2-methyl-2-heptenyl, 6-oxo-5-carbomethoxy-2-methyl-2-heptenyl, 6-oxo-2-methyleneheptyl, 6-oxo-2-methylene-5-carbomethoxyheptyl, 2,6-dimethyl-10-oxo-2,6-undecadienyl, 2,6-dimethyl-9-carbomethoxy-10-oxo-2,6-undecadienyl, 2-methyl-6-methylene-10-oxo-2-undecenyl and 2-methyl-6-methylene-9-carbomethoxy-10-oxo-2-undecenyl radical if appropriate in the form of acetal.

**Claims** for the contracting state AT

1. Process for preparing chlorinated ethylenic derivatives of general formula:

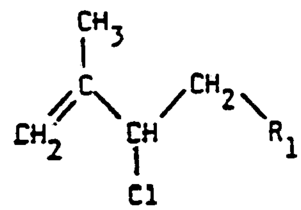

in which $R_1$ denotes a halogen atom or an acetyl or formyl radical if appropriate in the form of acetal, a hydroxy radical if appropriate in the form of ether or ester, an alkyloxycarbonyl radical whose alkyl part contains 1 to 4 carbon atoms, an alkyl radical containing from 1 to 12 carbon atoms and substituted by one or more identical or different radicals chosen from halogen atoms and acetyl and formyl radicals if appropriate in the form of acetal, a hydroxy radical if appropriate in the form of ether or ester or an alkyloxycarbonyl radical whose alkyl part contains 1 to 4 carbon atoms, an alkenyl radical containing 2 to 12 carbon atoms and one or more double bonds if appropriate substituted by one or more identical or different radicals, chosen from halogen atoms and acetyl and formyl radicals optionally in the form of acetal, a hydroxy radical if appropriate in the form of ether or ester or an alkyloxycarbonyl radical whose alkyl part contains 1 to 4 carbon atoms, $R_1$ being moreover able to denote a 3-sulpholenyl radical or a radical of general formula:

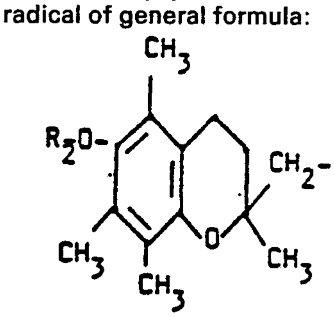

in which $R_2$ denotes a hydrogen atom or an acetyl radical, characterized in that chlorination of a product of general formula:

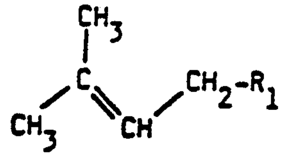

is carried out by means of chlorine by operating in a non-polar aprotic organic solvent at a temperature of between -10 and 100° C.

2. Process according to Claim 1, characterized in that the solvent is chosen from aliphatic saturated hydrocarbons, halogenated aliphatic hydrocarbons, cycloaliphatic hydrocarbons and aromatic hydrocarbons.

3. Process according to Claim 1, characterized in that it comprises operating at the reflux temperature of the reaction mixture, under reduced pressure if appropriate.

4. Process according to Claim 1, characterized in that the solvent is distilled off in an operation at constant volume.